# EUROPEAN PATENT APPLICATION

(11) **EP 1 391 732 A1**
(43) Date of publication of application: **25.02.2004**
(21) Application number: 02018908.0
(22) Date of filing: 23.08.2002
(51) Int. Cl.: G01N 33/68, C12Q 1/68, G01N 33/573

(54) **Caspase 10 as target for monitoring and treatment of diseases**

(71) Applicant: Deutsches Krebsforschungszentrum Stiftung des öffentlichen Rechts, 69009 Heidelberg (DE)
(72) Inventor: Walczak, Henning, 69120 Heidelberg (DE); Sprick, Martin, 69121 Heidelberg (DE)
(74) Representative: Weiss, Wolfgang, Dipl.-Chem. Dr.

(57) **Abstract**

The present invention relates to a method of monitoring and/or modulating disease-associated activatory procceses which are mediated by caspase-10 or caspase-10 isoforms.

## Description

The present invention relates to a method of monitoring and/or modulating disease-associated activatory procceses which are mediated by caspase-10 or caspase-10 isoforms. Further, the present invention relates to novel caspase-10 isoforms.

Apoptosis is an essential process during the development of the immune system and for the maintenance of T and B cell homeostasis. Besides tumor necrosis factor (TNF) itself, two other apoptosis-inducing members of the TNF family, CD95 (APO-1/Fas) ligand (CD95L), and TNF-related apoptosis-inducing ligand (TRAIL/APO-2L), have been shown to be involved in various immunological processes. The involvement of the CD95 receptor/ligand system in inflammation, activation-induced death of peripheral T cells, immune privilege, tumor evasion from the immune system, autoimmunity, and AIDS is well established (Krammer, Nature 407 (2000), 789-795; Nagata, Cell 88 (1997), 355-365). The functional analysis of the TRAIL receptor/ligand system has been complicated by the fact that a total of five different receptors for this cytokine have been identified (Griffith and Lynch, Curr. Opin. Immunol. 10 (1998), 559-563; Locksley et al., Cell 104 (2001), 487-501). Recently TRAIL has been shown to be functional in various processes of the immune system and tumor surveillance (Walczak and Krammer, Exp. Cell. Res. 256 (2000), 58-66). Of particular interest was the recent finding that TRAIL was necessary for NK-cell mediated suppression of liver-metastasis in a mouse tumor model (Smyth et al., J. Exp. Med. 193 (2001), 661-670; Takeda et al., Nat. Med. 7 (2001), 94-100).

The early biochemical events that result in apoptosis induction by TRAIL and CD95L have been studied by the analysis of the so-called death-inducing signalling complex (DISC) (Kischkel et al., EMBO J. 14 (1995), 5579-5588; Walczak and Sprick, Trends Biochem. Sci. 26 (2001), 452-453). Crosslinking of CD95 or the two apoptosis-inducing TRAIL receptors results in the recruitment of FADD (MORT1) and caspase-8 (Boldin et al., J. Biol. Chem. 270 (1995), 7795-7798; Chinnaiyan et al., Cell 81 (1995), 505-512) to the respective DISC (Bodmer et al., Nat. Cell Biol. 2 (2000), 241-243; Boldin et al., Cell 85 (1996), 803-815); Kischkel et al., Immunity 12 (2000), 611-620; Muzio et al., Cell 85 (1996), 817-827; Sprick et al., Immunity 12 (2000), 599-609). In a homotypic interaction, the death domain (DD) of FADD binds to the DD of CD95. The death effector domain (DED) of FADD in turn interacts with the DED of pro-caspase-8 and thereby recruits this proenzyme to the CD95 DISC (Medema et al., EMBO J. 16 (1997), 2794-2804). Pro-caspase-8 is proteolytically cleaved and thereby activated at the DISC. Activated caspase-8 then initiates the apoptosis-executing caspase cascade (Peter et al, Results Probl. Cell Diff. 23 (1999), 25-63).

Studies using cells from mice deficient in either FADD (Yeh et al., Science 279 (1998), 1954-1958) or caspase-8 (Varfolomeev et al., Immunity 9 (1998), 267-276) showed that these two proteins are essential for CD95L-induced apoptosis. However, Holler et al. and Matsumura et al. reported that human Jurkat cells deficient in caspase-8 underwent caspase-independent necrotic cell death after stimulation with highly active CD95L (Holler et al., Nat. Immunol. 1 (2000), 489-495); Matsamura et al., J. Cell. Biol. 151 (2000), 1247-1256). The molecular mechanisms responsible for this type of cell death remain largely unclear, although the molecule RIP has been proposed to be essential for CD95L induced necrosis (Holler et al. (2000), supra).

Until recently, the molecular explanation for a rare immunological disorder in children called autoimmune lymphoproliferative syndrome (ALPS) had been restricted to the CD95 receptor-ligand system (Fischer et al., Rev. Immunogenet. 2 (2000), 52-60; Straus et al., Ann. Intern. Med. 130 (1999), 591-601). However, recently two patients with severe ALPS were identified who neither carried mutations in the CD95 nor the CD95L gene. Comprehensive analysis of apoptosis associated genes in these patients revealed mutations in the caspase-10 gene. These mutations were shown to result in a disease termed ALPS II (Wang et al., Cell 98 (1999), 47-58). In this study it was proposed that TRAIL resistance of mature DC and activated peripheral T cells from ALPS II patients was due to the mutated non-functional caspase-10 and causative for the disease. Later, one of the mutations identified in this study has been found to be a common polymorphism in the Danish population (Gronbaek et al., Blood 95 (2000), 2184-2185). Although to date, besides the ALPS II patient identified by Wang et al., no other individual homozygous for this caspase-10 mutation has been described. This finding raised the question whether this mutation in caspase-10 alone is causative for the disease.

Caspase-10 (Mch4, FLICE-2) is the second DED-containing caspase besides caspase-8. It was identified by homology cloning (Fernandes-Alnemri et al., PNAS USA 93 (1996), 7464-7469; Vincenz and Dixit, J. Biol. Chem. 272 (1997), 6578-6583). The FLICE-2 and Mch4 isoforms represent different splice variants derived from the same gene. Later; two additional isoforms were identified (Ng et al., J. Biol. Chem. 274 (1999), 10301-10308). Mch4 is now known as Caspase-10a, FLICE-2 as caspase-10b, and the two recently identified variants as caspase-10c and caspase-10d. While the caspase-10a, -10b and -10d isoforms represent proteins that contain both, the large and small catalytic subunits, the caspase-10c variant represents a truncated form yielding a catalytically inactive molecule. While previous studies on the function of caspase-10 relied on overexpression of the different isoforms or catalytically inactive variants, no detailed study under native conditions with the endogenously expressed proteins has been conducted so far. Protein overexpression experiments are prone to artefacts, yielding at least a cautionary note to the results obtained in these systems. Further, the expression and distribution of the different caspase-10 isoforms has been unclear, as only mRNA levels have been investigated.

We therefore set out to study the endogenous expression levels of caspase-10 (i), whether it forms part of the TRAIL and the CD95 DISC under native conditions (ii), and whether caspase-8 and caspase-10 are functionally redundant or might fulfil different functions (iii).

Surprisingly and in contradiction to recently published scientific papers (Kischkel et al., J. Biol. Chem. 276 (2001), 46639-46646; Wang et al., PNAS USA 98 (2001), 13884-13888; Wang et al., Cell 98 (1999), 47-58) it was found that caspase-10 is not capable of replacing caspase-8, but plays an essential role in disease-associated activatory processes, particularly processes which are triggered by receptor crosslinking. Monitoring of such processes may be effected by determining of caspase-10 or caspase-10 isoforms. Prevention and/or therapy of such processes may be effected by modulating, i.e. increasing or inhibiting the amount and/or activity of caspase-10 or caspase-10 isoforms. Such modulation at an early step during receptor-induced signal transduction is associated with less side-effects than targeting further downstream-molecules in signalling pathways. Especially noteworthy is that a significant fraction of tumor cell lines have down-regulated caspase-10 expression implying a role of this caspase in a tumorigenesis.

Thus, a first aspect of the present invention is a method of monitoring and/or modulating disease-associated activatory processes comprising determining and/or influencing caspase-10 or caspase-10 isoforms in a cell or an organism.

The disease-associated activatory processes are preferably triggered by receptors, more preferably by receptor-crosslinking. For example, the activatory processes are triggered by non-apoptosis signals emanating from death receptors, particularly TRAIL-R1, TRAIL-R2, CD95, TNF-R1 (p55 TNF-R), TRAMP, DR6 or combinations thereof. On the other hand, the activatory processes are triggered by signals emanating from death or non-death receptor members of the TNF receptor family, particularly TNF-R, p60 (also known as p55 and TNFR1), p80 (also known as p75, TNFR2), Fas (CD95/APO-1), TRAIL receptor, LTβR, CD40, CD30, CD27, HVEM, OX40, TROY, EDAR, XEDAR, DCR3, AITR, 4-1 BB, DR3, RANK, TACI, BCMA, DR6, OPG and TRAIL-R1 (DR4), TRAIL-R2 (DR5), TRAIL-R3 (DcR1) and TRAIL-R4 (DcR2) and/or members of the TLR/IL-1 receptor family, particularly TLR-receptors TLR1-10, including TLR4 or TLR2 in complex with co-receptor CD14, heterodimers of TLR2/6 and TLR2/1, and IL-1 receptor family members IL-1R, IL-18R.

The disease may be selected from hyperproliferative, inflammatory and/or auto-immune diseases. Examples of inflammatory diseases are skin inflammatory diseases, e.g. granulocytosis or internal inflammatory diseases, e.g. septic shock and other inflammatory diseases. Examples of hyperproliferative diseases are tumors, e.g. neuroblastoma, intestine carcinoma such as rectum carcinoma, colon carcinoma, familiary adenomatous polyposis carcinoma and hereditary non-polyposis colorectal cancer, esophageal carcinoma, labial carcinoma, larynx carcinoma, hypopharynx carcinoma, tong carcinoma, salivary gland carcinoma, gastric carcinoma, adenocarcinoma, medullary thyroidea carcinoma, papillary thyroidea carcinoma, renal carcinoma, kidney paranchym carcinoma, ovarian carcinoma, cervix carcinoma, uterine corpus carcinoma, endometrium carcinoma, chorion carcinoma, pancreatic carcinoma, prostate carcinoma, testis carcinoma, breast carcinoma, urinary carcinoma, melanoma, brain tumors such as glioblastoma, astrocytoma, meningioma, neuroblastoma, medulloblastoma and peripheral neuroectodermal tumors, Hodgkin lymphoma, non-Hodgkin lymphoma, Burkitt lymphoma, acute lymphatic leukemia (ALL), chronic lymphatic leukemia (CLL), acute myeolis leukemia (AML), chronic myeloid leukemia (CML), adult T-cell leukemia lymphoma, hepatocellular carcinoma, gall bladder carcinoma, brohcial carcinoma, small cell lung carcinoma, non-small cell lung carcinoma, multiple myeloma, basalioma, teratoma, retinoblastoma, chorioidea melanoma, seminoma, rhabdomyosarcoma, craniopharyngeoma, osteosarcoma, chondrosacoma, myosarcoma, liposarcoma, fibrosarcoma, Ewing sarcoma and plasmocytoma. Examples of auto-immune diseases are ulcerative colitis, Crohn's disease, multiple sclerosis, rheumatoid arthritis, diabetes mellitus, pernicious anemia, autoimmune gastritis, psoriasis, Behcet's disease, Wegener's granulomatosis, Sarcoidois, autoimmune thyroiditis, autoimmune cophoritis, bullous pemphigoid, phemphigus, polyendocrinopathies, Still's disease, Lambert-Eaton myasthenia syndrome, myasthenia gravis, Goodpasture's syndrome, autoimmune orchitis, autoimmune uveitis, systemic lupus erythematosus, Sjogren's Syndrome and ankylosing spodylitis. Further suitable diseases are sepsis, haemorrhagic shock, Stevens-Johnson Syndrome, Toxic Epidermal Necrolysis, Spongiotic Dermatitis, Urticaria, Psoriasis, Lichen Planus, Lupus Erythematosis, Polyarteritis, Reiter's syndrome, Rheumatoid Arthritis, Scleroderma, Inflammatory Bowel Disease and Ankylosing Spondylitis.

One embodiment of the present invention relates to a method of monitoring a disease or a disease-associated activatory process as described above, comprising determining the presence, amount, localization and/or activity of caspase-10 or caspase-10 isoforms in a sample, e.g. in a body fluid or in a tissue section, particularly from a human patient. The determination of caspase-10 or caspase-10 isoforms may occur on the nucleic acid level, e.g. by detecting transcripts or cDNA derived from these transcripts. The determination on the nucleic acid level may comprise hybridization and optionally nucleic acid amplification procedures as known in the art. On the other hand, determination may occur on the protein level, e.g. by immunological methods, e.g. employing antibodies or antibody fragments which are specific for caspase-10 or caspase-10 isoforms. The determination of caspase-10 or caspase-10 isoforms may be carried out for the diagnosis and/or prognosis, or for the progression control and/or therapy control of a disease as described above.

A further embodiment of the present invention relates to the treatment of diseases or disease-associated activatory processes as described above, comprising modulating, i.e. increase or inhibiting the activity and/or amount of caspase-10 or caspase-10 isoforms in a cell or an organism. The cell is preferably a eukaryontic cell, more preferably a mammalian cell, e.g. a human cell. The organism is preferably a eukaryontic organism, more preferably a mammal, e.g. a human being. The activity and/or amount of caspase-10 or caspase-10 isoforms may be modulated on the nucleic acid level. For example, an inhibition may comprise administering anti-sense molecules, ribozymes or siRNA molecules directed against the caspase-10 mRNA. On the other hand, the amount and/or activity may be increased gene-therapeutic methods, wherein a nucleic acid molecule encoding caspase-10 operatively linked to a suitable expression control signal is administered e.g. by employing a viral or non-viral gene-transfer vector. The delivery of nucleic acid molecules capable of modulating caspase-1 activity may occur by known methods, e.g. by liposomes, liposome mediated transfer, viral vectors (retroviral, adenoviral), ballistic injection, dirct injection of naked DNA, cationic lipid complexed DNA, DNA in microparticles.

On the other hand, the amount and/or activity of caspase-10 or caspase-10 isoforms may be modulated on the protein level. For example, antibodies or antibody fragments directed against caspase-10 or caspase-10 isoforms, e.g. monoclonal antibodies, chimeric antibodies, humanized antibodies, human antibodies, single chain antibodies or fragments thereof, e.g. proteolytic fragments or recombinant fragments, or other binding proteins, such as anticalins, may be administered in a therapeutically effective dose in order to inhibit caspase-10 or caspase-10 isoforms. On the other hand, low molecular weight molecules, such a peptides or non-peptidic organic molecules which are capable of stimulating or inhibiting caspase-10 activity may be administered.

A preferred function for caspase-10 within the scope of the present invention is the activation of cytokinsecretion, either indirectly via intermediary signalling pathways or by direct proteolytic maturation. Cytokines secreted by cells after activation of caspase-10 might be pro- or anti-inflammatory, resulting in the recruitment of immune cells, e.g. in skin inflammatory diseases (granulocytosis etc.), septic shock and other inflammatory diseases. Regarding anti-tumor responses, loss of caspase-10 in the tumor can result in loss of secretion of cytokines and, hence, reduced infiltration of immune cells into the tumor. Thus, re-expression of caspase-10 in the tumor can be useful in order to allow for increased immune cell infiltration into the tumor. On the other hand loss of caspase-10 can serve as a diagnostic or prognostic marker in cancer.

A further preferred function for caspase-10 is the induction of direct cell bound signals, e.g. phosphatidylserine (PS) exposure on the outer leaflet of the plasma membrane following initiation of apoptosis, which lead to phagocytosis of cells stimulated by other cells (e.g. of the immune system). It has been shown that cells stimulated via death receptors are phagocytosed by neighboring cells before they completely underwent apoptosis. Silencing of phagocytosis signals could be advantageous for tumor cells, as they could survive low intensity pro-apoptotic signals (e.g. immune cell-mediated and/or chemo/radiotherapy-elicited) which do not lead to direct cell death but would require a following phagocytosis step. Caspase-10 activation may be involved in the translocation of PS from the inner to the outer leaflet of the plasma membrane and can thereby allow for the PS-mediated recognition of early apoptotic cells by phagocytes and can thereby allow for more efficient apoptosis. Thus, increasing caspase-10 activity is useful for the treatment of tumors and auto-immune disease (like e.g, ALPS II) where too little cells are phagocytosed. An increase in caspase-10 activity could be achieved e.g., by gene therapy with a caspase-10 expressing vector. Achieving a decrease in caspase-10 activity might be useful in disease situations where too many cells die or are phagocytosed. The decrease in caspase-10 activity can result in the survival of an otherwise phagocytosed cell. The decrease of caspase-10 activity could be achieved by e.g., an inhibitor of the enzymatic function of caspase-10, or an inhibitor of the recruitment of caspase-10 to the adaptor protein FADD/MORT1. Alternatively one could influence the expression levels of caspase-10 by inhibiting the expression of its RNA either anti-sense technology or by the application of caspase-10-specific small interfering RNA (siRNA).

Still a further preferred function of caspase-10 is being a transmitter of signals regulating proliferation, differentiation, and/or senescence. Caspase-10 is not directly implicated in death induction then it can play an essential role for other signalling pathways that have been reported to be activated after triggering receptors of the TNF receptor superfamily and other receptors (e.g. TLR) which have been implied (at least in part) to use caspases in order to transmit their signals. These pathways include but are not limited to activatory signalling pathways including kinase pathways like e.g., pathways involving MAP kinase and related kinases, Jun kinases and related kinases, PKB (Akt), and kinase pathways that result in the activation of NF-kB and/or its family members. In that respect, caspase-10 mRNA has been shown to be induced by bacterial products (Nielsen et al, J. Immunol. 167 (2001), 5231-5239) and heat shock (Navita et al., Int. J. Radiation Oncology Biol. Phys. 53 (2002), 198-196). Inhibition of caspase-10 could possibly block these activatory pathways. It could be useful to block the activatory functions of caspase-10 in order to block diseases which are caused by an over-stimulation of e.g. the immune system. These include but are not limited to auto-immune diseases, sepsis, hemorrhagic shock.

Modulators of caspase-10 or caspase-10 isoforms are usually administered as a pharmaceutical composition which contains the active ingredients and optionally further pharmacologically acceptable carriers, diluents and/or adjuvants. The pharmaceutical composition is administered in a therapeutically effective dose, i.e. a dose which is suitable for the treatment of a specific condition. The dose may vary according to the type of active ingredient (nucleic acid, antibody or low-molecular weight compound) and the type and severity the disease to be treated. A suitable dose may be determined by the skilled person without undue effort.

The pharmaceutical composition may be administered alone or in combination with other active ingredients which are known for the treatment of a specific disease as described above.

A further aspect of the present invention relates to a method of identifying and/or characterizing compounds which are capable of modulating disease-associated activatory processes, particularly processes associated with the diseases as described above. The method comprises determining if a test compound is capable of influencing the amount and/or activity of caspase-10 or caspase-10 isoforms. In one embodiment, the method is a screening method for indentifying new modulators of caspase-10 or caspase-10 isoforms. In a further embodiment the method is carried out for characterizing the effects and/or side-effects of already known therapeutically effective compounds on caspase-10. The method may be a cellular assay, wherein a suitable host cell, particularly a mammalian cell is provided which expresses or overexpresses caspase-10 and wherein the effect of a test compound on the cell, e.g. on morphological characteristics of the cell, is determined. On the other hand, the method may be a molecular assay, wherein the effect of a test compound on purified or partially purified caspase-10 is determined. The effect of a test compound on caspase-10 may comprise a binding assay and/or a caspase-10 activity assay. In a further preferred embodiment the effect of a test compound on a transgenic animal expressing or overexpressing caspase-10 may be determined.

The present invention relates to nucleic acid encoding caspase-10 and caspase-10 isoforms and polypeptides encoded thereby. Preferred caspase-10 isoforms are
caspase-10d (GenBank Acc. No. NM_ 032977)
caspase-10c (GenBank Acc. No. NM_ 032976) and
caspase-ba (GenBank Acc. No. NM_ 001230).

Further, the invention relates to partical sequences thereof, e.g. having a length of at least 15 nucleotides, preferably at least 50 nucleotides.

Furthermore, the invention relates to mutated sequences of caspase 10 isoforms, e.g. conservative mutations, which do not alter the polypeptide sequence and/or function, like e.g. polymorphisms such as caspase-10d V410I (valine at position 410 exchanged by isoleucin).

Further, the present invention is explained in more detail by the following figures and examples.

### Description of figures

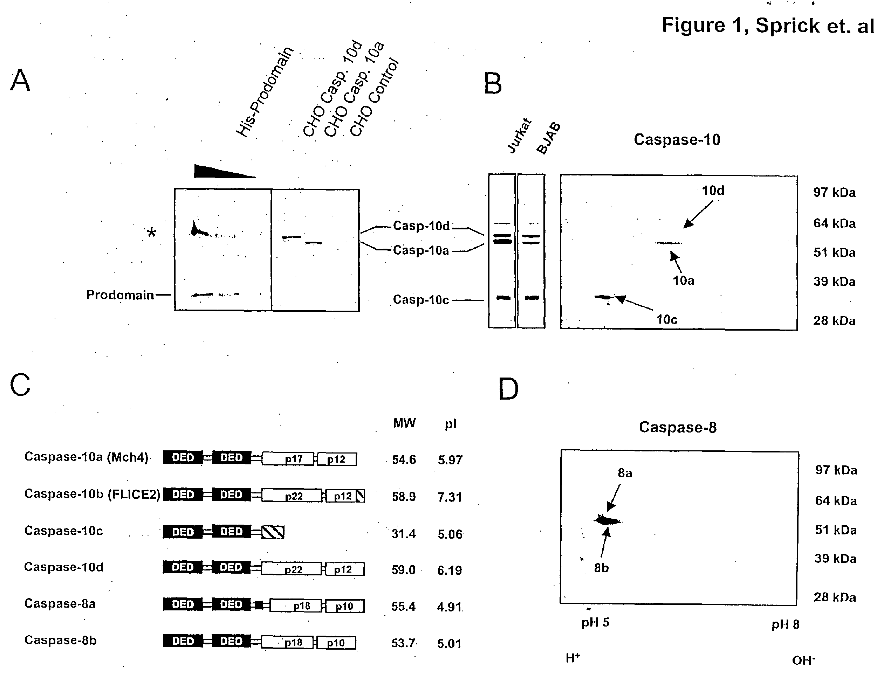

**Fig. 1.** Three isoforms of caspase-10 are expressed in BJAB and Jurkat cell lines. (A) Specificity of the caspase-10 antibody was determined by blotting decreasing amounts of bacterially expressed His-tagged caspase-10 prodomain or lysates from CHO-cells transfected with expression plasmids for human Caspase-10d, -10a or a control plasmid. The asterisk denotes a co-purified protein resulting from inefficient termination of translation. (B) Three endogenous isoforms of caspase-10 can be detected in lysates from BJAB and Jurkat cells. 10 µg proteins from the indicated cell lines were resolved on SDS page, blotted and detected with the caspase-10 antibody. Lysates from BJAB cells were resolved on 2D gels spanning pH 5- pH 8, blotted and subsequently detected with caspase-10 antibody. The pl and MW of the large caspase-10 isoforms correspond to caspase-10d and caspase-10a, the small protein to the pl and MW of caspase-10c. (C) Overview of the calculated MW and pl of the reported caspase-10 and caspase-8 isoforms. (D) As a control for the pl determination, the 2D-gel blot was reprobed with an antibody to caspase-8, showing the caspase-8 isoform at the expected positions.

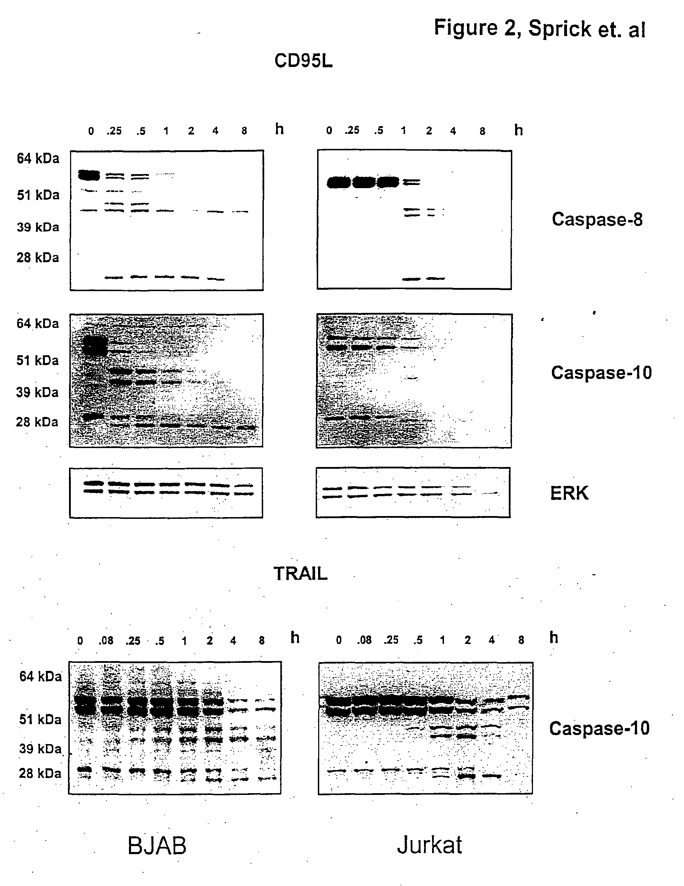

**Fig. 2.** Caspase-10 is processed in TRAIL- and CD95L-sensitive cell lines after stimulation with the respective ligands. Cells were stimulated for the indicated time periods with TRAIL (1 µg/ml) or CD95L (500 ng/ml). Lysates were prepared, and analysed by western blot with antibodies to caspase-8 and caspase-10. As a control, the blot containing lysates from cells stimulated with CD95L was reprobed with an antibody to ERK. Cleavage of all caspase-10 isoforms can be observed as early as 15 to 30 minutes for stimulation with CD95L (upper panel) or TRAIL (lower panel), respectively. Cleavage of caspase-10 leads to the appearance of two intermediate products, p47 and p43 and a smaller band corresponding to the prodomain at 25 kDa. The cleavage pattern is similar to that observed for caspase-8 which is included as a control. The lower amount of ERK control observed after 8h of stimulation is the result of almost complete cell death observed at this time point.

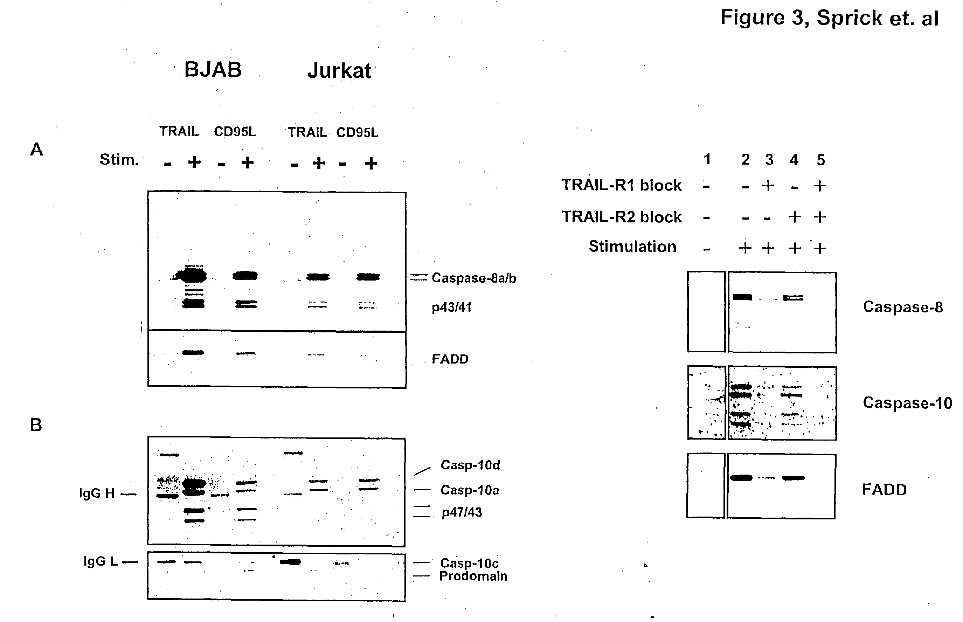

**Fig. 3.** Caspase-10 is recruited to the native TRAIL and CD95 DISCs. Precipitation of the DISC complexes which form upon stimulation with TRAIL and CD95L was performed in BJAB and Jurkat cells. The resulting precipitated protein complexes were separated by SDS PAGE, and analysed by western blot. (A) To control for DISC formation, the western blots were probed for the known DISC components caspase-8 and FADD showing recruitment of these proteins to the TRAIL and the CD95 DISC in BJAB and Jurkat cells. (B) The DISC complexes were also analysed for the presence of caspase-10, showing that caspase-10 is recruited to the TRAIL DISC and the CD95 DISC in both cell lines tested. All three caspase-10 isoforms associate with both complexes. In addition, the intermediate products p47, p43 and the fragment corresponding to the prodomain, p25, are also associated with the CD95 and the TRAIL DISCs. The light chain of the M2 anti-FLAG antibody migrates at the same MW as the caspase-10 isoform. Correlation of heavy and light chain levels reveals caspase-10c association with crosslinked receptors.

(C) Caspase-10 associates with homomeric TRAIL-R1 and TRAIL-R2 complexes. Differential TRAIL DISC analysis was carried out by blocking either TRAIL-R1 or TRAIL-R2 with monoclonal antibodies (10 µg/ml for 20 min at 25°C) against the respective receptors before DISC analysis was performed. The resulting complexes were separated by SDS-PAGE and analysed by western blot. While precipitation of the unstimulated receptors shows no association of the proteins analysed (lane 1), under conditions where both receptors are stimulated (lane 2) caspase-10 associates with both, TRAIL-R2 complexes (lane 3) and TRAIL-R1 complexes (lane 4). Blockage of both receptors completely abolishes DISC formation (lane 5).

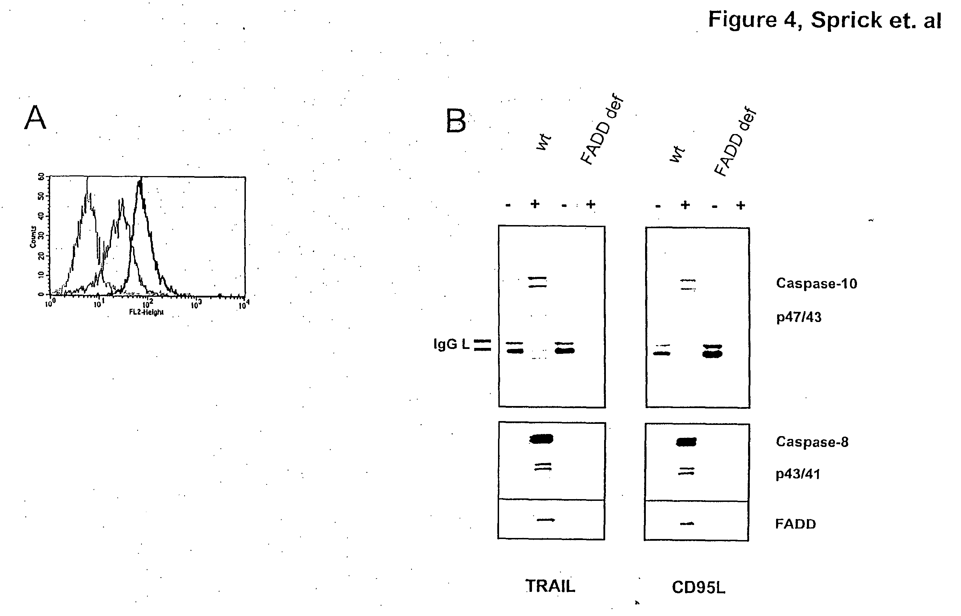

Fig. 4. FADD is the adapter protein for caspase-10 recruitment to TRAIL-R1, TRAIL-R2 and CD95. (A) FACS stain of the FADD deficient cell line FADDdef TR1/TR2. A surface stain for TRAIL-R1 (thin line) and TRAIL-R2 (thick line) shows expression of both TRAIL-R1 and TRAIL-R2. Dotted line, IgG control. (B) DISC analysis was performed with TRAIL and CD95L on wild type and FADDdef TR1/TR2 Jurkat cells. Western blot analysis of the precipitated protein complexes shows that caspase-8, caspase-10 and FADD are efficiently recruited to the TRAIL DISC and the CD95 DISC in the parental wild type (wt) cells. In the absence of the adapter protein FADD in the cell line Jurkat FADDdef TR1/TR2 (FADD def) caspase-10 as well as caspase-8 are neither recruited to the TRAIL-DISC nor to the CD95-DISC.

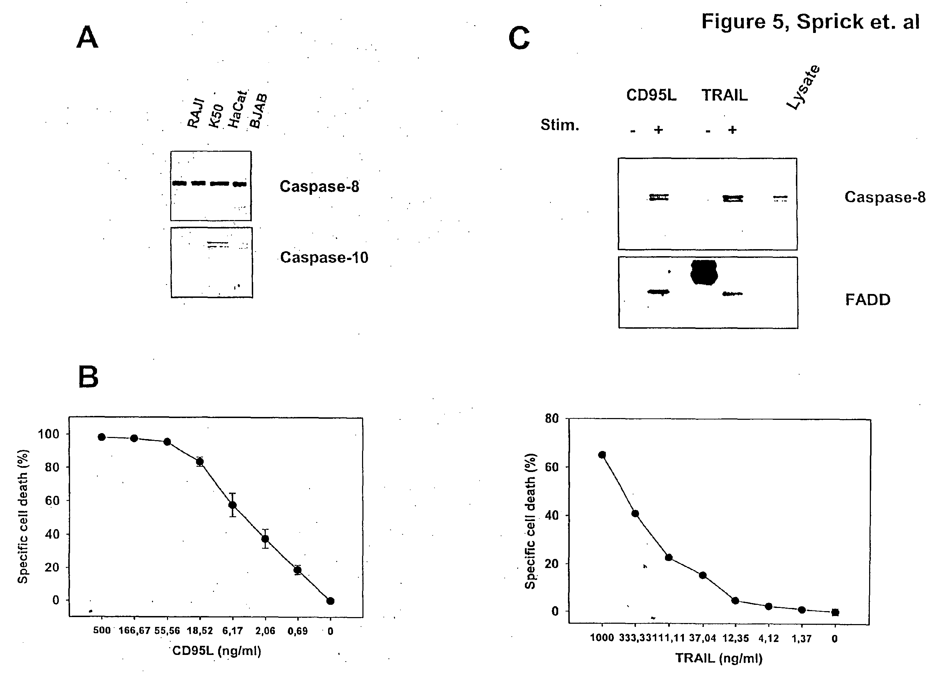

Fig. 5. Caspase 10 is not required for for TRAIL and CD95L mediated cell death and activation of caspase-8. (A) Western blot of lysates of different cell lines shows that some cell lines do not express detectable levels of caspase-10. (B) Cells from the cell line K50 were incubated in CD95L or TRAIL at the concentrations indicated. Cell death was measured after 16 h by PI exclusion and FSC/SSC analysis. Both ligands induce cell in the absence of detectable levels of caspase-10. (C) Analysis of the TRAIL and CD95 DISCs in K50 cells shows that caspase-8 is recruited and activated at both DISC complexes in the absence of caspase-10. The DISCs were precipitated and analysed as in the experiment presented in Figure 4B.

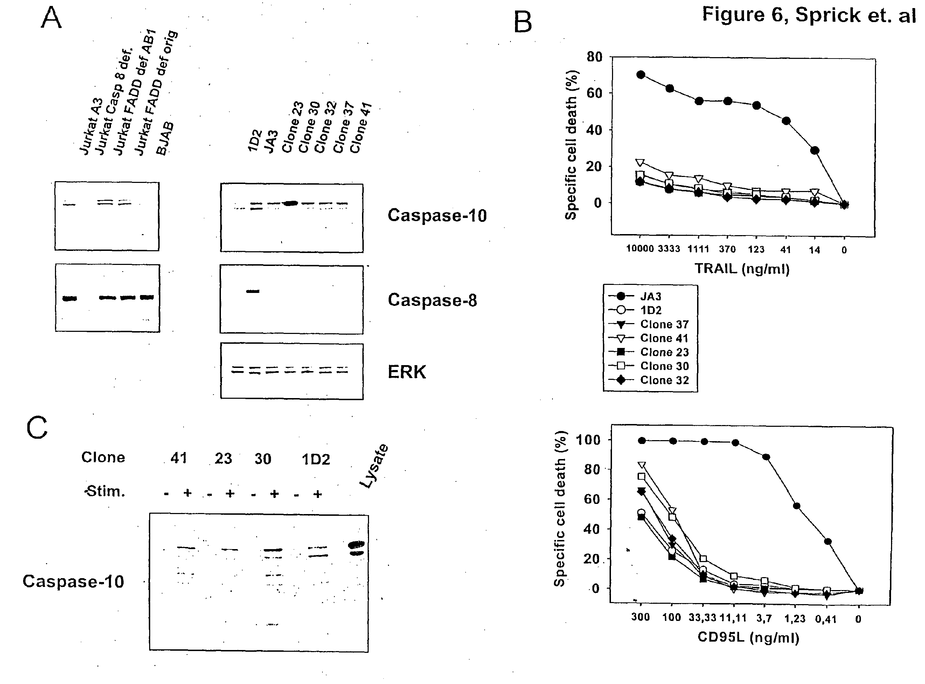

Fig. 6 Caspase 10 can not functionally substitute loss of caspase-8. (A) Comparison of the expression levels of caspase-10 in different cell lines and clones stably transfected with caspase-10. Cell lysates from the cell lines indicated were resolved by SDS Page and blotted. The blots were probed with antibodies against caspase-10 and caspase-8. (B) High expression levels of caspase-10 do not sensitize caspase-8 deficient cell lines to TRAIL or CD95L induced apoptosis. Different cell lines were incubated with either TRAIL (upper panel) or CD95L (lower panel) at the concentrations indicated. Cell death was assessed after 24 h by measuring the drop in FSC/SSC and PI exclusion. (C) Caspase-10 is recruited to and activated at the CD95 DISC in the absence of caspase-8. Cells were stimulated with CD95L at 100 ng/ml for 15 minutes. After lysis, the DISC complexes were precipitated with an antibody against CD95 (anti APO-1), resolved on SDS and blotted. The blots were probed for Caspase-10, showing recruitment of caspase-10 in caspase-8 deficient cell line 1 D2 and in the caspase-10 transfected caspase-8 deficient cell lines. Clone 41, 23 and 30. Caspase-10 is recruited to the CD95 DISC in all cell lines even in the absence of caspase-8. Also, in all cases caspase-10 is activated in these complexes as indicated by the appearance of the p47/p43 fragments. L denotes the lane containing control lysate from unstimulated clone 41.

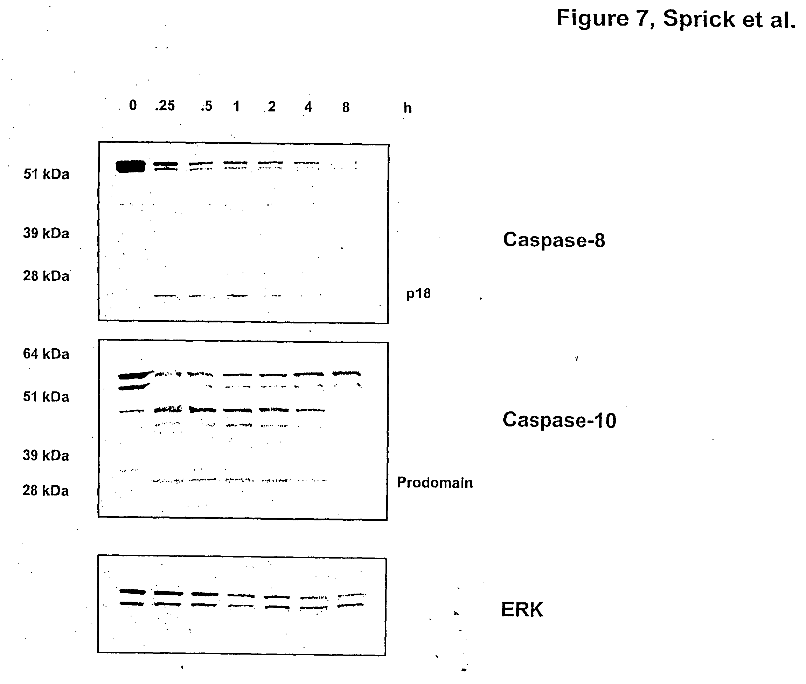

**Fig.7** Caspase-10 is expressed and rapidly processed in primary T cells after stimulation with CD95L. Activated primary T cells were stimulated with CD95L (500 ng/ml) for the time periods indicated, lysed and subjected to western blot analysis. Processing of caspase-10 and caspase-8 can be detected simultaneously at 15 min. after stimulation as indicated by the appearance of the p18 subunit of caspase-8 (upper panel) or the processed prodomain of caspase-10 (lower panel), respectively. The cleavage fragments of caspase-10 p47, p43 and the prodomain, p25, correspond to the pattern detected in BJAB and Jurkat cell lines (Fig. 2).

### Example

### 1. MATERIALS AND METHODS

### 1.1 Cell Lines

The human B cell line BJAB, the human T cell line Jurkat A3, the caspase-8 deficient Jurkat clone, the Jurkat FADDdef clone and its derivative, the TRAIL-R1-expressing Jurkat FADDdef TR1/TR2 clone (Juo et al., Cell Growth. Diff. 10 (1999), 797-804) were maintained in RPMI 1640 (Gibco-BRL, Karlsruhe, Germany) containing 10% fetal calf serum (GIBCO-BRL) The CHO cell line was maintained in Ham_s F12 medium with 10% fetal calf serum.

### 1.2 Cloning of Caspase-10 isoforms

Plasmids coding for the caspase-10 isoforms were obtained by amplification of the different caspase-10 open reading frames by PCR with Pfu polymerase. Primer sequences for amplification were: CASP10UP: 5'-atgaaatctcaaggtcaacattggtattcc-3', CASP10LOW: 5'-ctatattgaaagtgcatccaggggcacagg-3'. PCR fragments were ligated into pEFV5His-TOPO vector (Invitrogen, Karlsruhe, Germany) for eukaryotic expression. The stop codon contained in the caspase-10 cDNA was included to generate an untagged version of caspase-10. Insert containing vectors were fully sequenced and verified to contain the desired products without mutations.

### 1.3 Bacterial expression of the caspase-10 prodomain

The caspase-10 prodomain was amplified by PCR from a vector containing full length caspase-10d and subcloned into pCRT7NT-Topo (Invitrogen, Karlsruhe, Germany). His-tagged prodomain was expressed in E.coli BL21 and purified on a Ni²⁺ column under denaturing conditions.

### 1.4 Transient transfection of CHO cells.

CHO cells were transiently transfected with the expression plasmids for caspase-10a, -10d or a control vector with Fugene transfection reagent according to the manufacturer's (Roche, Mannheim, Germany) instructions. 24h after transfection, cell lysates were collected and analysed.

### 1.5 Stable transfection of caspase-10 and TRAIL-R1 in Jurkat caspase-8 deficient cells

First, a caspase-8-deficient cell line stably expressing TRAIL-R1 was generated by transfecting the parental caspase-8 deficient cell line with the expression vector pCDNA3 Hygro-coding for TRAIL-R1. After initial selection in Hygromycin B (200 µg/ml), single clones were obtained by two rounds of limiting dilution cloning. Clones were analysed by surface staining for expression of TRAIL-R1 and one clone, denoted clone 1D2, was chosen.

Caspase-8 deficient cell lines stably overexpressing caspase-10d were generated by transfecting the caspase-8 deficient cell line with the pEFV5His-TOPO-Casp-10d vector using Xtreme-GeneQ2 (Roche, Mannheim). After initial selection in Blasticidin-S (10 µg/ml), single clones were obtained by limited dilution cloning. To exclude the introduction of mutations in caspase-10 during the generation of the stable clones, the cDNA encoded by the plasmid was re-isolated from the single clones, sequenced and found to be without mutations.

### 1.6 2D Gel analysis

For 2D analysis cells were directly lysed for 1h in 2D sample buffer (7M Urea, 2M Thiourea, 4%CHAPS, 2mM Tris-Butylphosphine, supplemented with complete protease inhibitors (Roche, Mannheim, Germany). Subsequently, the samples were clarified by centrifugation (50000g, 30 minutes). For the first dimension, protein samples were diluted in 2D rehydration buffer (2D sample buffer supplemented with bromophenol blue, 1% ampholytes pH 5-8 (Bio-Rad, München, Germany, 10% (v/v) glycerol). The first dimension isoelectric focussing (IEF) was performed with ReadyStrip precast IPG strips pH 5-8 essentially according to the manufacturer's instructions (Bio-Rad, München, Germany). After electrofocussing, the strips were incubated two times for 15 minutes in equilibration buffer (50mM Tris pH 8.8, 6M Urea, 30% Glycerol, 2% (w/v) SDS, 10 mg/ml DTT). The second dimension was run on precast 4-12% NuPage Bis-Tris gels (Invitrogen, Karlsruhe, Germany) with MOPS running buffer and subsequently gels were blotted onto nitrocellulose membrane.

### 1.7 Establishment of the cell line FADDdef TR1/TR2

The TRAIL-R1- and -R2-expressing FADD-deficient Jurkat cell line TR1/2 (clone 6D2-1 D2) was derived from the original Jurkat FADDdef cell line by transfection with an expressionplasmid coding for the complete TRAIL-R1 open reading frame. The transfected pool was subjected to two rounds of limiting dilution cloning, choosing the cell line with the highest TRAIL-R1 surface expression as evaluated by FACS staining with a TRAIL-R1-specific monoclonal antibody (HS102).

### 1.8 Antibodies and Reagents

Monoclonal antibodies (mAb) against FADD were purchased from Transduction Laboratories (San Diego, CA). Anti-Caspase 10 mAb (Clone 4C1) was from MBL International (Watertown, MA) and anti ERK polyclonal serum from St. Cruz. Antibodies which were tested for reactivity with caspase-10 on western blots of lysates of transfected CHO cells and found to be non-specific were: PharMingen #67041N, Upstate Biotechnology #06-836, Oncogene Sciences anti-caspase-10 #Ab-2. The monoclonal antibody MAB834 from R&D Systems recognized the transfected caspase-10, but was unsuited for analysis of endogenous proteins in cell lysates as it strongly cross-reacted with several proteins. The mAb anti-FLICE C15 recognizes the p18 subunit of caspase-8 (Scaffidi et al., J. Biol. Chem. 272 (1997), 26953-26958). Anti FLAG M2 was purchased from Sigma (Taufkirchen, Germany). The monoclonal antibodies specific for the different TRAIL receptors were generated by immunizing mice with the respective TRAIL receptor-Fc fusion proteins. Specificity of the antibodies was confirmed by staining cells transfected with expression plasmids for TRAIL-R1 to -R4. Anti-TRAIL-R1 (clone HS101) and anti-TRAIL-R2 (clone HS201), both of the mlgG1 isotype, were used for FACS staining and receptor blockage. The antibodies are available from Alexis (Gruenberg, Germany). Horseradish peroxidase (HRPO)-conjugated goat anti-mouse IgG1, IgG2b and mouse K light-chain specific polyclonal antibodies (pAb) were obtained from Southern Biotechnology Associates (Birmingham, AL). Flag-CD95L was from Alexis (Gruenberg, Germany). Flag-TRAIL was expressed as previously described (Keogh et al., FEBS Lett. 471 (2000), 93-98). All other chemicals used were of analytical grade and purchased from Merck (Darmstadt, Germany) or Sigma Chemical Co. (Taufkirchen, Germany).

### 1.9 FACS analysis

Cells (5x106) were incubated with mAbs against TRAIL-R1 (HS102), TRAIL-R2 (HS201), or control mlgG1 at 10 µg/ml followed by biotinylated secondary goat anti-mouse antibodies (Southern Biotechnology Associates, CA) and Streptavidin-PE (Pharmingen, Hamburg, Germany). Surface staining was analysed on a FACS-Calibur (Becton Dickinson, Heidelberg, Germany).

### 1.10 Preparation of cell lysates

Cells were harvested by centrifugation at 300 x g for 10 min at 4 °C, washed twice with ice cold PBS and lysates were prepared by resuspending the resulting cell pellets in 100 µl lysis buffer per 1 x107 cells (30 mM Tris-HCI pH 7.5, 120 mM NaCI, 10 % Glycerol 1% Triton X-100) supplemented with Complete™ protease inhibitors (Roche Diagnostics, Mannheim, Germany) according to the manufacturer's instructions. After 30 min incubation on ice, the lysates were centrifuged once at 15,000 x g at 4 °C to remove nuclei. For the lysis of primary T cells the protease inhibitor concentration was increased five times.

### 1.11 Western blot analysis

For Western blot analysis the resulting postnuclear supernatants or DISC precipitates were supplemented with 2-fold concentrated standard reducing sample buffer (2 x RSB). Subsequently, lysate containing 20 µg of protein as determined by the BCA method (Pierce, Rockford, IL) or proteins eluted from beads after ligand affinity immunoprecipitation were separated on 4-12% NuPage Bis-Tris gradient gels (Novex, San Diego, CA) in MOPS buffer according to the manufacturer's instructions. After protein transfer onto nitrocellulose membranes (Amersham Pharmacia Biotech, Freiburg, Germany) by electroblotting, membranes were blocked with 5% non-fat dry milk (NFDM) in PBS/Tween (PBS containing 0,05% Tween-20) for at least 1 h, washed with PBS/Tween, and incubated in PBS/Tween containing 3% non-fat dry milk (NFDM) and primary antibodies against caspase-10, FADD or caspase-8 (1:10-diluted C15 hybridoma supernatant. After 5 washes for 3 min each in PBS/Tween the blots were incubated with HRPO-conjugated isotype-specific secondary antibody diluted 1:20,000 in PBS. After washing 5 times for 3 min with PBS/Tween the blots were developed by enhanced chemoluminescence (ECL) using SuperSignal West Dura substrate following the manufacturer's protocol (Perbio Science, Bonn, Germany). For stripping, blots were incubated in 50 mM glycine HCI 500 mM NaCI pH 2.3 for 20 min at room temperature. Subsequently, blots were washed two times for 10 min in PBS/Tween and blocked again.

### 1.12 DISC analysis

We performed DISC analysis by using FLAG-tagged TRAIL (FLAG-TRAIL) or Flag-tagged CD95L (Flag-CD95L) in combination with Streptavidin beads (Pierce). For ligand affinity precipitation 1-5 x 10⁷ cells were used per sample. Cells were washed with 50 ml RPMI medium at 37 °C and subsequently incubated for the indicated time periods at 37 °C and a cell density of 5 x 10⁷/ml in the presence of 1 µg/ml FLAG-TRAIL pre-complexed with 2 µg/ml anti-FLAG M2 for 15 minutes, or, for the unstimulated control, in the absence of FLAG-TRAIL. In the case of differential TRAIL receptor DISC analysis we preincubated the cells with 10 µg/ml TRAIL-R1 and/or TRAIL-R2-blocking mAbs for 15 min before stimulation with FLAG-TRAIL. DISC formation was stopped by addition of at least 15 volumes of ice-cold PBS. Cells were then washed twice with 50 ml ice cold PBS before cell lysates were prepared. Cells were lysed by addition of 1 ml lysis buffer. The resulting protein complexes were precipitated from the lysates by co-incubation with 20 µl Protein G Beads (Roche, Mannheim, Germany) for 2-4 hours on an end-over-end shaker at 4 °C. For the precipitation of the non-stimulated receptors, FLAG-TRAIL plus M2 was added to the lysates prepared from non-stimulated cells at 200 ng to control for protein association with non-stimulated receptor(s). Ligand affinity precipitates were washed 5 times with lysis buffer before the protein complexes were eluted from the beads by addition of 15 µl 2 x standard reducing sample buffer. Subsequently, proteins were separated on SDS-PAGE before detection of DISC components by western blot analysis.

### 2. RESULTS

### 2.1 Caspase-10 is expressed in three isoforms

The role of caspase-10 in CD95- and TRAIL-mediated apoptosis has been controversial. At least four splice variants of caspase-10 have been reported. Three of the reported caspase-10 isoforms contain the large and the small subunits of caspase-10 and yield potentially active enzyme. The fourth variant, caspase-10c, codes for a truncated form that lacks the catalytic subunits and, thus, represents a catalytically inactive form of caspase-10 reminiscent of the CAP3 form of caspase-8. To investigate the potential role of caspase-10 during TRAIL- and CD95L-mediated apoptosis we first set out to identify a caspase-10-specific antibody. Amongst a panel of commercially available antibodies that we tested only one antibody specifically recognized bands at 55 and 59 kD in Chinese hamster ovary (CHO) cells transfected with expression plasmids encoding recombinant caspase-10a and -10d, respectively (Fig. 1A). The epitope recognized by this antibody is localized in the death-effector-domain-containing prodomain as the antibody also detects the recombinantly expressed His-tagged prodomain (Fig. 1A). All splice variants of caspase-10 reported thus far contain this prodomain. Therefore, this antibody should recognize all known isoforms of caspase-10 while all other antibodies we tested neither reacted with the recombinant prodomain nor with overexpressed caspase-10a or -10d in CHO cells (data not shown).

Having identified a specific antibody for caspase-10 we investigated which isoforms of caspase-10 are expressed in the human Burkitt's lymphoma cell line BJAB and the human T cell line Jurkat. Two large isoforms of caspase-10 (p55/p59) were detected in the lysates derived from both cell lines (Fig. 1B), reminiscent of the two large isoforms of caspase-8. At about 28 kD an additional isoform of caspase-10 was detected. To determine to which of the published isoforms the individual bands correspond we performed two-dimensional (2D) gel electrophoretic analysis (Fig. 1B). The calculated molecular weights and isoelectric points (pl) for the different isoforms of caspase-10 are shown in Fig. 1C. As a control, the same blot was calibrated by detection of caspase-8 showing that the 2D-determined pl of the p55 and p53 caspase-8 isoforms exactly matched their predicted pl of 4.9 and 5.0, respectively (Fig. 1D). Determination of the pl of the two expressed large isoforms of caspase-10 revealed that they represent caspase-10d (p59) and caspase-10a (p55). This analysis further excluded that one of them could represent caspase-10b (calculated pl 7.3). As the pl of the 28 kD band matched the pl of caspase-10c this protein spot most likely corresponded to this isoform of caspase-10.

### 2.2 Caspase-10 is cleaved early during CD95- and TRAIL-induced apoptosis

Caspase-8 has been shown to be the apoptosis-initiating caspase in the CD95 and the TRAIL DISCs and, thus far, caspase-8 has been thought to be the earliest caspase to become cleaved during death receptor-mediated apoptosis. Knowing that caspase-8 is cleaved during TRAIL- and CD95L-induced apoptosis we next analysed whether caspase-10 might also be processed during death receptor-mediated apoptosis and whether this cleavage occurs prior to, concomitantly with, or following caspase-8 cleavage (Fig. 2). During TRAIL- and CD95L-induced apoptosis the two large isoforms of caspase-10 are cleaved resulting in two intermediate cleavage products of caspase-10a and -10d, p43 and p47, respectively (Fig. 2). Further cleavage of these two intermediates results in the appearance of a 25 kD band representing the prodomain of caspase-10. Also, the 28kD caspase-10c is cleaved during TRAIL- and CD95-induced apoptosis. Thus, caspase-10 is present in both BJAB and Jurkat cells in three different isoforms and all of these isoforms are cleaved during TRAIL- and CD95-mediated apoptosis. Depending on cell type and stimulus this cleavage was apparent between 15 and 30 minutes after onset of the incubation with TRAIL or CD95L concomitantly with the appearance of caspase-8 cleavage in the cytoplasm.

### 2.3 Caspase-10 is recruited to the CD95 and the TRAIL DISC in BJAB and Jurkat cells

Like caspase-8, caspase-10 contains the tandem DEDs necessary for homotypic interaction with the adapter protein FADD, making recruitment of caspase-10 to death receptor-bound FADD-containing complexes possible. Caspase-8 is recruited to the CD95, the TRAIL-R1 and the TRAIL-R2 signalling complexes after ligand-induced stimulation of the respective receptors in a FADD-dependent fashion. As results concerning protein-protein interactions that solely rely on transient protein expression are prone to artefacts we analysed the receptor complexes under native conditions. We compared the death-inducing signalling complexes (DISCs) that form after stimulation with CD95L and TRAIL in both, Jurkat and BJAB cells and tested whether caspase-10 is recruited to these complexes (Fig. 3). To control for efficiency of DISC formation we analysed recruitment of caspase-8 and FADD, two already known components of the TRAIL and CD95 DISC (Fig. 3A). Whereas none of the proteins analysed were bound to the non-stimulated receptors we observed a stimulation-dependent recruitment of FADD and caspase-8 to CD95 and the TRAIL death receptors, as described before (Bodmer et. al. (2000), supra; Kischkel et al., J. Biol. Chem. 276 (2001), 46639-46646; Sprick et al. (2000), supra). For caspase-10 the analysis revealed ligand-induced recruitment of all three expressed caspase-10 isoforms to the native TRAIL DISC and the native CD95 DISC in both cell types (Fig. 3B). Like for caspase-8, the products of the first activation step of caspase-10a and -10d (p43/p47) were also associated with the TRAIL and the CD95 DISC. These two forms represent the prodomain with the p17 subunit of caspase-10a and -10d, respectively after separation of the p12 subunit by proteolytic cleavage. In addition, the prodomain is also apparent in both DISCs (Fig. 3B, lower panel).

### 2.4 Caspase-10 is recruited to the TRAIL-R1 and the TRAIL-R2 DISC

So far no biochemical differences in recruitment of adaptor and effector proteins between TRAIL-R1 and TRAIL-R2 have been identified. To investigate whether caspase-10 is recruited selectively to one of the apoptosis-inducing TRAIL receptors, we performed differential DISC analysis for TRAIL-R1 and TRAIL-R2 on BJAB cells (Fig. 3C). Either of the two receptors was blocked by incubation with a monoclonal antibody specific for TRAIL-R1 or TRAIL-R2 before stimulation of the other receptor by the ligand. Efficiency of inhibition of the receptors was demonstrated by complete inhibition of FADD and caspase-8 recruitment upon simultaneous TRAIL-R1 and TRAIL-R2 blockage. The analysis of the resulting complexes shows that both, caspase-10a and -10d were recruited to both receptors independently of the other receptor.

### 2.5 FADD is necessary for the recruitment of caspase-10 to the CD95, the TRAIL-R1 and the TRAIL-R2 DISC

FADD has been shown to be required for the recruitment and subsequent activation of caspase-8 to the TRAIL-R2 and the CD95 DISC. To test whether FADD is also required for recruitment of caspase-10 to the CD95, TRAIL-R1 and TRAIL-R2 DISC we investigated the respective protein complexes in cells that lack FADD but express all three receptors (Fig. 5). As the original FADD-deficient Jurkat cells only expressed TRAIL-R2 (Bodmer et al. (2000), supra; Sprick et al. (2000), supra) we established a stable subclone of this cell line transfected with a TRAIL-R1 expression plasmid. This clone which we named Jurkat FADDdef -TR1/2 expressed TRAIL-R1 and TRAIL-R2 on its surface (Fig. 5A). The protein complexes that form upon stimulation with CD95L and TRAIL are shown in Fig. 5B. While the CD95 and TRAIL DISC were efficiently formed in the parental Jurkat cells by incubation of these cells with CD95L and TRAIL, respectively, the absence of FADD resulted in failure of the crosslinked receptors to recruit caspase-8 and caspase-10. Thus, FADD is not only necessary for recruitment and activation of caspase-8 but also for recruitment and activation of caspase-10 at the TRAIL and CD95 DISC.

### 2.6 Caspase-10 is not necessary for death-receptor mediated apoptosis and caspase-8 activation at the DISC

As both, caspase-8 and caspase-10 are present in the DISC complexes, it could be possible that both are needed for apoptosis to be initiated at the death receptors CD95 and the TRAIL receptors. We noted that several cell lines, including the Burkitt's lymphoma cell line BL60 and the CD95-transfected BL60 subclone K50, do not express detectable levels of caspase-10. Although K50 cells are devoid of detectable levels of caspase-10 (Fig. 5A upper panel), stimulation with CD95L or TRAIL resulted in normal apoptosis induction (Fig. 5A). In addition, caspase-8 is recruited to both, the CD95 and the TRAIL DISCs in this cell line and activated as indicated by the presence of the cleavage products, p43/41 in the DISC (Fig. 5B). Thus, caspase-10 is dispensable for activation of caspase-8 at the DISC and concomitant apoptosis induction.

### 2.7 Caspase-10 can not functionally substitute caspase-8, although it is recruited to the DISC in the absence of caspase-8.

It has been previously shown by us and others that Jurkat cells deficient in caspase-8 are defective in apoptosis induction after stimulation with CD95L or TRAIL. However, analysis of expression of caspase-10 in this cell line revealed that the levels of caspase-10 are substantially reduced compared to the parental cell line (Fig. 6A). To test whether caspase-10 can induce apoptosis in the absence of caspase-8, we established stable clones that expressed caspase-10d in the absence of caspase-8. To expand our analysis of the TRAIL DISC to TRAIL-R1, we first generated a caspase-8 deficient cell line stably transfected with a TRAIL-R1 expression plasmid, as the Jurkat caspase-8def cell lines normally express only TRAIL-R2. A resulting clone was transfected with an expression plasmid for caspase-10. Several independent caspase-10 expressing clones were chosen for analysis. The amounts of caspase-10 expressed in these clones ranged from relatively low levels to amounts highly exceeding the amount of caspase-10 present in the parental cell line, the parental Jurkat caspase-8def and also, the amounts present in primary T cells (not shown). Expression of the TRAIL receptors and CD95 was comparable in the different cell lines as determined by surface staining (not shown). We next analysed TRAIL sensitivity of these cell lines in comparison to the parental clones. Surprisingly, apoptosis induction was not significantly higher in caspase-10 transfected versus non-transfected caspase-8 deficient cell lines (Fig. 6A upper panel).

Stimulation with CD95L leads to induction of cell death in the caspase-8-deficient cell lines, although much higher amounts of CD95L are needed comparing caspase-8-deficient cell lines to the caspase-8-expressing control cell line (Figure 6B lower panel). Recently, cell death induction by CD95L in caspase-8-deficient Jurkat cells was described (Heller et al. (2000), supra; Matsumura (2000), supra). However, as seen for stimulation with TRAIL, overexpression of caspase-10 does not lead to a sensitisation to CD95L induced apoptosis when compared to the parental caspase-8-deficient cell line. The inability of caspase-10 to substitute for caspase-8 could be due to its inability to be activated at the DISC complexes in the absence of caspase-8. However, analysis of the DISC shows that caspase-10 is recruited to and activated at the CD95 DISC also in the absence of caspase-8 (Fig. 6C). These results show that although caspase-10 is recruited to and activated at the DISC, it can not functionally substitute for caspase-8 in apoptosis induction.

### 2.8 Caspase-10 is expressed in primary human T cells and is cleaved upon CD95-induced apoptosis of pre-activated human T cells

Mutations in caspase-10 have been shown to result in the human autoimmune lymphoproliferative syndrome type II (ALPS II) that is characterized by a defect in T cell and dendritic cell apoptosis (Wang et al. (1999), supra). These results prompted us to test whether caspase-10 might also play a role in apoptosis induction in primary cells. We, therefore, analysed whether caspase-10 is cleaved during CD95-induced apoptosis of pre-activated human T cells. This analysis showed that caspase-10a, -10d and -10c were present in lysates obtained from these cells and that all caspase-10 isoforms were cleaved following CD95 stimulation (Fig. 7). The observed cleavage pattern was essentially identical to the pattern observed in BJAB and Jurkat cells (Fig. 2). The proteolytic activation of caspase-10 during apoptosis of activated T cells is indicative of a role for this caspase in the transmission of signals emanating from death receptors not only in transformed cell lines but also in primary cells of the immune system.

## Claims

1. A method of monitoring and/or modulating disease-associated activatory process comprising determining and/or influencing the amount and/or activity of caspase-10 or caspase-10 isoforms in a cell or an organism.

2. The method of claim 1 wherein the activatory processes are triggered by receptor-crosslinking.

3. The method of claim 1 or 2, wherein the activatory processes are triggered by non-apoptosis signals emanating from TNF receptor family members, particularly from death receptors, including but not limited to TRAIL-receptor 1 (DR4), TRAIL-receptor 2 (DR5), CD95 (APO-1, Fas), TNF-R1 (p55 TNF-R), TRAMP (DR3), DR6 or combinations thereof.

4. The method of claims 1 to 3, wherein the activatory processes are triggered by signals emanating from non-death receptor members of the TNF receptor family and/or from death receptor members of the TNF receptor family and/or members of the TLR receptor family.

5. The method of any one of claims 1 to 4, wherein the disease is selected from hyperproliferative, inflammatory and auto-immune diseases.

6. The method of claim 5, wherein the disease is an inflammatory disease selected from skin inflammatory diseases and septic shock.

7. The method of claim 5, wherein the disease is a hyperproliferative disease selected from tumors.

8. The method of claim 5, wherein the disease is an auto-immune disease.

9. The method of any one of claims 1 to 8 comprising monitoring the presence, amount, localization and/or activity of caspase-10 or caspase-10 isoforms in a sample.

10. The method of claim 9, wherein caspase-10 or caspase-10 isoforms are determined on the nucleic acid level.

11. The method of claim 9, wherein caspase-10 or caspase-10 isoforms are determined on the protein level.

12. The method of any one of claims 1 to 8 comprising modulating the amount and/or activity of caspase-10 or caspase-10 isoforms in a cell or an organism.

13. The method of claim 12, wherein the amount and/or activity of caspase-10 or caspase-10 isoforms is modulated on the nucleic acid level.

14. The method of claim 12, wherein the amount and/or activity of caspase-10 or caspase-10 isoforms is modulated on the protein level.

15. A pharmaceutical composition comprising a modulator of the activity of caspase-10 or caspase-10 isoforms as an active ingredient and optionally pharmaceutically acceptable carriers, diluents and/or adjuvants.

16. A method of identifying and/or characterizing compounds for the modulation of disease-associated activatory processes comprising determining if a test compound is capable of influencing the activity of caspase-10 or caspase-10 isoforms.
